(19) Europäisches Patentamt
European Patent Office
Office européen des brevets

(11) Publication number: 0 270 885 A1

(12) **EUROPEAN PATENT APPLICATION**

(21) Application number: 87116995.9

(22) Date of filing: 17.11.87

(51) Int. Cl.4: C07F 9/65 , C07D 473/18 , C07D 473/34

(30) Priority: 18.11.86 US 932111

(43) Date of publication of application: 15.06.88 Bulletin 88/24

(84) Designated Contracting States: AT BE CH DE ES FR GB GR IT LI LU NL SE

(71) Applicant: Bristol-Myers Company
345 Park Avenue
New York New York 10154(US)

(72) Inventor: Webb II, Robert R.
42 Harrison Road
Guilford Ct. 06437-1412(US)
Inventor: Martin, John C.
40 Brookside Place
Cheshire, Ct. 06410(US)

(74) Representative: Kinzebach, Werner, Dr.
Patentanwälte Reitstötter, Kinzebach & Partner Sternwartstrasse 4 Postfach 86 06 49
D-8000 München 86(DE)

(54) **Synthesis of purin-9-ylalkylenoxymethyl phosphonic acids.**

(57) An improved process for the preparation of purin-9-ylalkylenoxymethyl phosphonic acids, novel nucleotide analogs, typified by (S)-9-(3-hydroxy-2-phosphonylmethoxypropyl)adenine, also known as (S)-HPMPA. The improved process employs novel intermediates, is shorter and more efficient by virtue of eliminating large scale isomer separations and ion exchange chromatography, and also is applicable to guanine derivatives as well as to adenine derivatives.

EP 0 270 885 A1

Xerox Copy Centre

Background of the Invention

This invention is an improved, more labor efficient process for the synthesis of novel purine nucleotide analogs (I)

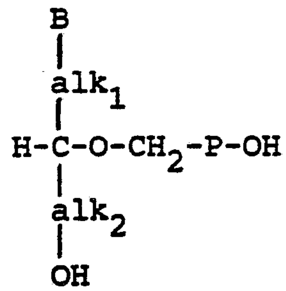

I

wherein $alk_1$ and $alk_2$ are independently selected from $C_{1-4}$ alkylene; and B is the base adenin-9-yl or guanin-9-yl. Compounds of Formula I are useful as selective broad-spectrum antiviral agents. An example of one subclass of these compounds, wherein B is adenin-9-yl, has been disclosed by De Clercq, et al., in Nature, 1986, 323, pp. 464-467. The specific compound disclosed by DeClercq is (S)-9-(3-hydroxy-2-phosphonylmethoxypropyl)adenine, also known as (S)-HPMPA, whose structure is shown below as (a),

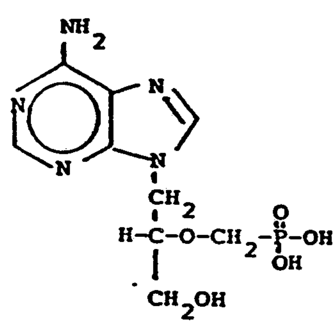

(S)-HPMPA

(a)

and was disclosed earlier by Holy, et al., Nucleic Acids Research, Symposium Series No. 14, 1984 pp. 277-278.

A synthetic method for preparing (S)-HPMPA and its isomeric analogs as well as certain intermediate compounds such as 9-(2,3-dihydroxypropyl)adenine (DHPA) was disclosed in the UK Patent Application of Holy, et al., GB 2,134,907A, published 8/22/84.

This prior art synthesis is shown in Scheme I as follows:

**Scheme I**

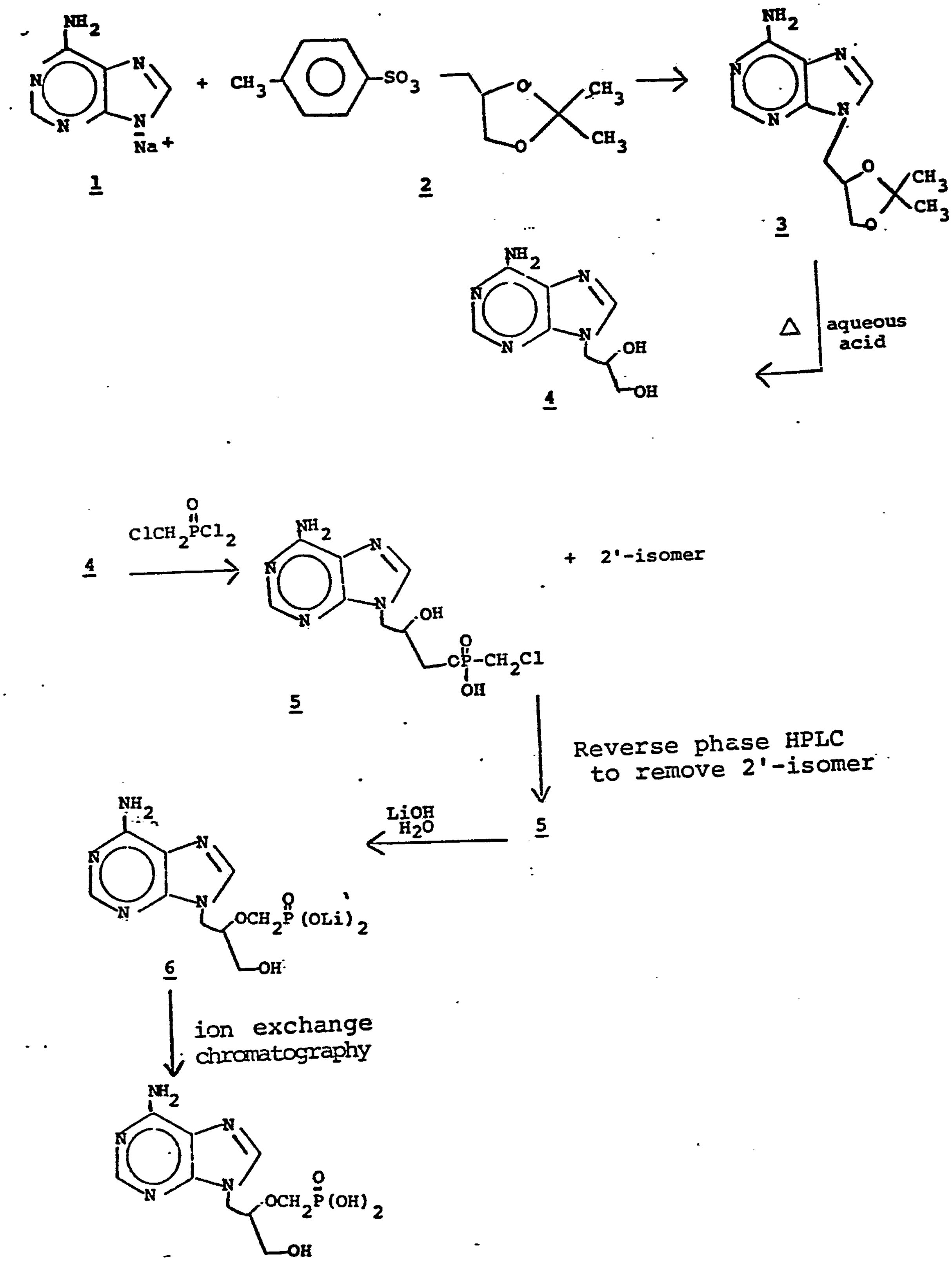


1 2 3 4 4 5 5 6

HPMPA

As shown in Scheme I, alkylation of the sodium salt of adenine 1 with isopropylidene-D-glycerol- γ -tosylate 2 gives the acetonide intermediate 3 which is heated with aqueous acetic acid yielding 9-(2,3-dihydroxypropyl)adenine 4, also known as DHPA. DHPA 4 is then treated with chloromethylphosphonic dichloride to give a mixture of the chloromethyl phosphonate intermediate 5 and its 2'-isomer. Separation of these isomers can be accomplished by reverse-phase high pressure liquid chromatography (HPLC) eluting with 0.1M aqueous phosphoric acid. Phosphonate 5 is then treated with concentrated aqueous lithium hydroxide giving the rearranged dilithium salt of HPMPA 6. A purification by de-salting ion exchange column chromatography is required to obtain the desired phosphonic acid form of HPMPA.

It would be advantageous to eliminate the need for large scale isomer separation and subsequent ion exchange chromatography required in the prior art synthetic method described above, both of which are cumbersome procedures especially on a large scale. We have conceived a synthesis of purine nucleotide analogs of formula I which proceeds from the readily obtainable dihydroxypropyl purine intermediate, e.g., DHPA 4 and employs the synthetic "isolation" of the secondary hydroxyl in the alkylene chain

by means of appropriate protecting groups. Use of protecting groups, particularly those with steric bulk such as the ones utilized in the instant process are known to be useful in preparation of protected derivatives of ribonucleosides and nucleotides. As examples of representative references disclosing protection of base amino groups and sugar hydroxy groups by the triphenylmethyl ("trityl") class of protective agent see:

1) Martin, et al., J. Med. Chem, 29, 1389 (1986).
2) Schaller, et al., JACS, 85, 3821 (1963);
3) Hata, et al., Bull. Chem. Soc. Japan, 55, 2949-2955 (1982).

While these references show that both sugar-O'-oxygen and purine-amino nitrogen atoms have been protected with trityl-type protecting groups in nucleoside and nucleotide synthetic applications, there is no teaching or suggestion in the prior art which would predict their selective applicability as in the process of the instant invention.

## Summary of the Invention

This invention relates to an improved synthetic process which can be adapted for large-scale preparation of purin-9-ylalkylenoxymethyl phosphonic acids which are novel nucleotide analogs. The instant process starts with readily

synthesized 9-(2,3-dihydroxypropyl)purines. The subject improved process offers advantages in economies of both material and labor costs by virtue of eliminating large scale isomer separations and subsequent ion exchange chromatography and in being applicable to guanine as well as adenine purine bases.

## Detailed Description of the Invention

The improved process comprising the present invention is illustrated in the synthetic flow chart shown below as Scheme II.

Scheme II

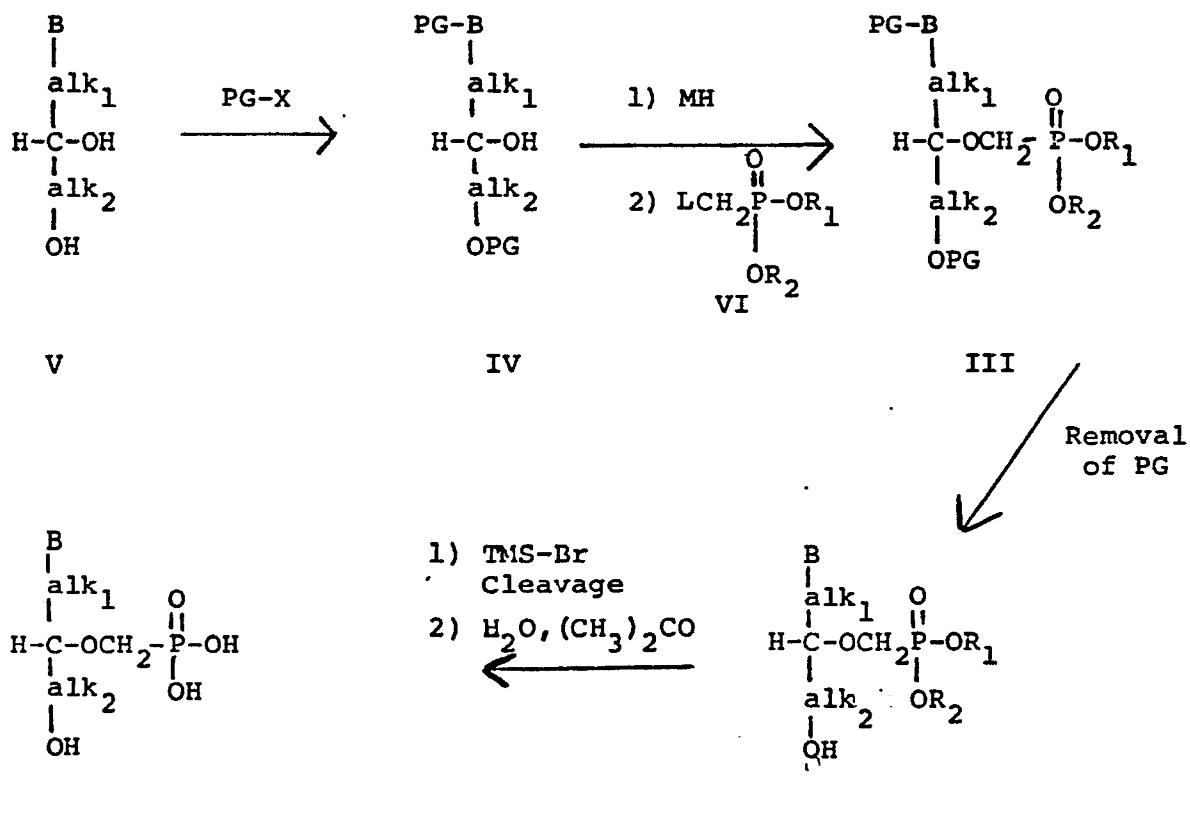


V IV VI III

I II

In another aspect, this invention comprises novel intermediates, namely, the novel di-protected group-intermediate, IV, and the novel dialkylphosphonylmethyl intermediate, III, produced in the practice of the process according to this invention. Particularly, with regards to the aspect of the invention relating to novel intermediates, the invention comprises those intermediates wherein the base, B, in the intermediates represented by formulas IV and III, respectively, is guanin-9-yl.

In Scheme II, B is a purine base selected from adenin-9-yl or guanin-9-yl; $alk_1$ and $alk_2$, independently, are selected from $C_{1-4}$ alkylene; PG is a selective protecting group having steric bulk and X is a leaving group moiety selected from halide groups; MH is a metal hydride; $R_1$ and $R_2$, independently, are selected from $C_{1-6}$ alkyl; and L is an organic leaving group.

Step 1 of Scheme II involves protecting the amino group moiety of a purine base selected from adenine and guanine and the terminal hydroxy group of the starting diol, V, to obtain a protected purin-9-yl alkylene diol, IV. The protecting group PG selected is one whose steric and electronic properties are such that the protecting group is selectively bonded to the purine amino group and terminal

alkylene chain hydroxy group at rates which favor no bonding with the secondary hydroxyl group located in the interior of the alkylene chain. Examples of protecting groups of this type are the triphenylmethyl class of protecting groups, including unsubstituted triphenylmethyl ("trityl") groups and substituted triphenylmethyl groups with substitution on one or more of the phenyl rings. It is to be understood that there are protecting groups additional to trityl possessing the requisite electronic and steric properties which allow their use in the instant process and utilization of these additional protecting groups are considered part of the instant process. Examples of such additional protecting groups include p-methoxyphenyldiphenylmethyl and di-(p-methoxyphenyl)phenylmethyl to name but a representative few. The symbol X of reagent PG-X represents a leaving group selected from halide groups including chloride, bromide and iodide, more preferably chloride and bromide, most preferably, chloride. Reaction conditions for introduction of protecting groups would be well known to one skilled in the art of organic synthesis and would be readily obtainable from the chemical literature by one skilled in the pertinent art. In general, protecting groups are introduced in reactions carried out in reaction-inert solvents usually containing an excess of a basic reagent

such as triethylamine and pyridine, whose function is to react with and remove, that is, "scavenge", the leaving group species and hydrogen ion which are liberated as the reaction proceeds. Examples of suitable inert solvents include dimethylformamide (DMF), tetrahydrofuran (THF) and dimethylsulfoxide (DMSO) to name but a few. In a preferred process, step 1 involves the reaction of at least two equivalents of triphenylmethyl chloride for each equivalent of the purin-9-yl alkylene diol intermediate, V, used. The reaction solvent is dimethylformamide containing triethylamine to scavenge the HCl generated in the reaction. A small amount of N,N-dimethylaminopyridine is also added to enhance the reaction. Typical yields of IV in step 1 are on the order of about 65% and above. Intermediate products of Formula IV in the preferred process can exist in two main subclasses and these are shown below as IVa and IVb.

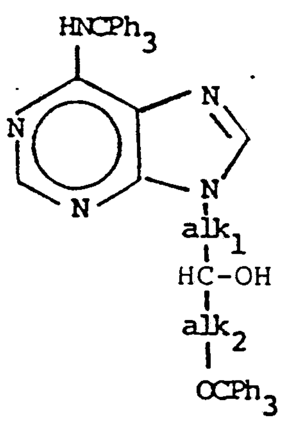

IV a

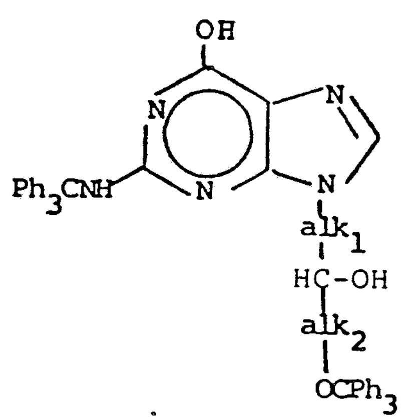

IV b

Step 2 of the process involves introduction of the phosphonylmethyl moiety at the secondary hydroxy group. This is accomplished by treating the protected, more particularly, the N,O-diprotected purin-9-ylalkylene diol, IV, with a metal hydride followed by reaction with a dialkyl phosphonate derivative, VI. This successful phosphonylmethylation was surprising and unexpected because the secondary hydroxyl group of the diprotected group-intermediate, IV, is sterically blocked. Preferred metal hydrides to be used in the process are the alkali metal hydrides such as lithium hydride, sodium hydride, and potassium hydride. The most preferred hydride is sodium hydride. The dialkyl phosphonate reagent, VI, is selected from a group of reagents wherein L is an organic leaving group selected from those well known to one skilled in the art of organic synthesis. Representative of such organic leaving groups are toluenesulfonate (tosylate), methanesulfonate (mesylate) and trifluromethanesulfonate (triflate) groups. The tosylate leaving group is a preferred leaving group for the present process. Thus, treatment of IV with sodium hydride in a nonprotic, reaction-inert solvent such as those mentioned above in the description of Step 1, for example, tetrahydrofuran, followed by addition of tosyloxymethyl dialkyl phosphonate gives the desired di-protected phosphonate intermediate of Formula III. Step 2 proceeds in relatively high yields giving typically about

a 90% yield of the compound III from intermediate IV.

Step 3 involves removal of the protecting groups from intermediate III. This can be done by either heating intermediate compound III in acidic media or by means of mild hydrogenolysis. The reaction conditions for removal of protecting groups would be familiar to one skilled in the art. A preferred method for removal of the triphenylmethyl-type of protecting group is by heating in acidic media. A more preferred method of removal comprises heating III in 80% aqueous acetic acid at about 80$^{\circ}$C for about one-half to one hour. Standard triphenylmethyl protecting group removal is a high yield process typically giving yields of about 85% of the dialkyl phosphonomethyl intermediate, II.

The last step of the process involves the trialkylsilylhalide (or halotrialkylsilane) mediated ester dealkylation of the dialkyl phosphonate moiety to the phosphonic acid structure of the product of Formula I. A preferred method of cleavage in this last step in the current process consists of reacting the dialkyl phosphonate intermediate, II, with the halotrialkylsilane reagent to effect cleavage followed by addition of water for conversion to the phosphonic acid I.

In a more preferred method of cleavage of II, intermediate II is reacted with a halotrimethylsilane, the most preferred

halo atoms being bromo and iodo. The product of Formula I may be readily isolated in its zwitterionic hydrate form by the further addition of acetone to the preferred cleavage and hydrolysis reaction medium.

In another more preferred embodiment, this last step of the process involves the use as the halotrialkylsilane reagent of halotrimethylsilane with the halo being selected from bromo and iodo and wherein there is included after reaction with the halotrimethylsilane and before the addition of water the further step of removing volatile reactants, by-products and solvents.

Another advantange of the overall process as outlined in Scheme II is that the stereochemistry introduced in the starting diol compound of Formula V is maintained throughout thereby allowing specification of the desired stereo chemistry in the product by selecting the appropriate starting stereoisomer.

The synthesis of purin-9-yl alkyleneoxymethyl phosphonic acids as representative of the improved process according to this invention is preferably carried out as a series of four steps proceeding from the starting material (the intermediate diol compound of Formula V) to the desired product of Formula I. The steps comprising the process are as follows:

(1) Reacting the appropriate purin-9-yl alkylene diol V with a protective group precursor compound of Formula PG-X, wherein PG is a selective protecting group having steric bulk, such as triphenylmethyl, and X is a leaving group moiety selected from halide groups, to give the di-protected group-intermediate IV;

(2) Treating IV with an alkali metal hydride and then with a dialkyl phosphonate reagent of Formula VI to yield the dialkylphosphonylmethyl intermediate III;

(3) Removing the protecting groups PG from III by standard protecting group removal process such as hydrolysis in acidic media or mild hydrogenolysis to give a de-protected dialkylphosphonylmethyl intermediate II; and

(4) Cleaving the phosphonate ester alkyl groups from intermediate compound II by trialkylsilylhalide (or halotrialkylsilane) mediated ester dealkylation to give the desired product I.

A preferred embodiment of this invention is a process for preparing (S)-9-(3-hydroxy-2-phosphonylmethoxypropyl)-B wherein B is a purine base selected from adenin-9-yl or guanin-9-yl, which comprises the consecutive steps of:

1) reacting 9-(S)-(2,3-dihydroxypropyl)B with chloro-triphenylmethane to give N,3'-O-bis-triphenylmethyl-(S)-9-(2',3'-dihydroxypropyl)B;

2) treating N,3'-O-bis-triphenylmethyl-(S)-9-(2',3'-dihydroxypropyl)B with sodium hydride and then with tosyloxymethyl diethylphosphonate to obtain N,3'-O-bis-triphenylmethyl-2'-O-(diethylphosphonylmethyl)-(S)-9-(2',3'-dihydroxypropyl)B;

3) heating N,3'-O-bis-triphenylmethyl-2'-O-(diethylphosphonylmethyl)-(S)-9-(2',3'-dihydroxypropyl)B in aqueous acetic acid to obtain 9-(2'-O-diethylphosphonylmethyl)-(S)-(2',3'-dihydroxypropyl)B; and

4) treating (2'-O-diethylphosphonylmethyl)-(S)-9-(2'3'-dihydroxypropyl)B with bromotrimethylsilane and then with water followed by acetone to give 2'-O-phosphonylmethyl-(S)-9-(2',3'-dihydroxypropyl)B.

Description of the Specific Embodiments

The process of this invention is illustrated in greater detail by the following examples directed to certain preferred embodiments of the hereinabove described process steps. These examples however should not be construed as limiting the scope of the present invention in any way. In examples which follow, used to illustrate the foregoing process, temperatures when unspecified are expressed, as in the foregoing, in degrees centigrade. Melting points are uncorrected. The proton nuclear magnetic resonance (PMR) spectral characteristics refer to chemical shifts (δ)

expressed in parts per million (ppm) versus tetramethylsilane (TMS) as reference standard. The relative area reported for the various shifts in the proton NMR spectral data corresponds to the number of hydrogen atoms of a particular functional type in the molecule. The nature of the shifts as to multiplicity is reported as broad singlet (bs), singlet (s), broad multiplet (bm), multiplet (m), doublet (d), doublet of doublets (dd), triplet (t), or quartet (q). Abbreviations employed are DMSO-$d_6$ (perdeuterodimethylsulfoxide), $CDCl_3$ (deuterochloroform) and are otherwise conventional. The infrared (IR) spectral descriptions include only absorption wave numbers ($cm^{-1}$) having functional group identification value. The IR determinations were employed using potassium bromide (KBr) as diluent. All compounds gave satisfactory elemental analysis. Examples 1-4 concern adenin-9-yl as the base and the remaining examples concern guanin-9-yl as the base.

Example 1

Step 1)

N6,3'-O-Bis-(Triphenylmethyl)-(S)-9-(2',3'-dihydroxy)propyl adenine (N,O-ditrityl DHPA; IV)

A suspension of DHPA (9-(2',3'-dihydroxypropyl)-adenine), 27.50 g, 0.131 mol) in dry dimethylformamide (200 mL) was treated with chlorotriphenylmethane (80.70 g, 0.289 mol), triethylamine (92 mL, 0.655 mol) and N,N-dimethylamino pyridine (1 g), and the resulting mixture heated to 80$^{o}$ for 12 hours. The resulting solution was then cooled, the volatile reactants and by-products were removed under reduced pressure, and the remaining residue was partitioned between ethyl acetate and water. The combined ethyl acetate layers were dried over magnesium sulfate, and concentrated under reduced pressure. The oil remaining was purified by chromatography over silica gel, eluting with 1:1 ethyl acetate-hexane. The appropriate fractions were combined and concentrated under reduced pressure to produce about a 65% yield of ditrityl-DHPA IV as a colorless foam. An analytical sample was obtained from $CCl_4$/hexane as an amorphous crystalline solid, mp. 230-231$^{o}$C,

$^{1}$H NMR(360MHz, $CDCl_3$, $Me_4Si$) 7.93(s, 1H), δ7.66(s, 1H), 7.27(m, complex, 30H), 7.08(s, 1H), 4.40(d, J=14Hz, 1H),

4.30(dd, J=7,14Hz, 1H), 4.15(d, J=7Hz, 1H), 3.26(dd, J=7Hz, 1H), 2.94(dd, J=7Hz, 1H), 1.25(s, 1H, exchanges with $D_2O$); Analysis: ($C_{46}H_{39}N_5O_2$) C,H,N.

Example 2

Step 2)

N6,3'-O-Bis-(Triphenylmethyl)-2'-O-(diethylphosphonylmethyl)-(S)-9-(2',3'-dihydroxypropyl)adenine III

A solution of ditrityl-DHPA (IV 9.12g,0.0137 mol) in dry tetrahydrofuran (30 mL) was treated, under argon, with sodium hydride (0.6 g, 0.0150 mol, 60% by weight dispersion in oil). The resulting suspension was stirred for 15 minutes, and then tosyloxymethyldiethylphosphonate (4.0 g, 0.0137 mol) dissolved in tetrahydrofuran (10 mL) was added. The resulting mixture was stirred for 14 hours at room temperature under argon, at which time thin layer chromatographic analysis of the mixture indicated the reaction was complete. Brine was then added, and the mixture extracted exhaustively with ethyl acetate. The combined ethyl acetate layers were dried over magnesium sulfate, and concentrated under reduced pressure. The oil obtained was chromatographed over silica eluting first with 1:1 ethyl acetate-hexane, and then with 90:5:5 ethyl acetate-hexane-methanol. The appropriate fractions were

combined and concentrated under reduced pressure to yield 10.3gm (90%) of the phosphonate III as a colorless foam. An analytical sample was obtained from ethyl acetate-hexane by slow dilute crystallization to yield amorphous crystals, mp. 88-89°C, $^1H$ NMR(360MHz, $CDCl_3$, $Me_4Si$) δ7.97(s, 1H), 7.82(s, 1H), 7.39(dd, J=3.5, 11Hz, 6H), 7.32(dd, J=3.5, 11Hz, 6H), 7.26(m, complex, 18H), 6.90 (s, 1H), 4.33(ABqd, J=7, 14Hz, 2H), 3.94(m, complex, 6H), 3.60(dd, J=11, 14Hz, 1H), 3.22(ABqd, J=3.5,11Hz, 2H), 1.25(t, J=7Hz, 3H), 1.17(t, J=7Hz, 3H); Analysis: ($C_{51}H_{50}N_5O_5P$) C,H,N.

Example 3

Step 3)

2'-O-(Diethylphosphonylmethyl)-(S)-9-(2',3'-dihydroxy)propyl)adenine (diethyl-S-HPMPA; II)

A solution of 2 g (0.00236 mol) ditritylphosphonylmethyl DHPA, III was heated on a steam bath with 80% aqueous acetic acid. After 1/2 hour, thin layer chromatographic analysis indicated the reaction was complete. The volatile reactants and by-products were removed by co-distillation with 4 successive volumes of absolute ethanol under reduced pressure, and finally with 2 volumes of toluene similarly. The solid residue remaining was purified by chromatography over silica gel eluting with 10% methanol in methylene

chloride to yield 0.754gm (89%) of diethyl-S-HPMPA II as a crystalline solid, mp. 154°; $[\alpha]_D^{25} = -35°$ (ethanol, C=.26). UV: λ max 260, $^1$H NMR(200MHz, $CDCl_3$, $Me_4Si$) δ8.2(s, 1H), 7.9(s, 1H), 5.8(br s, 2H), 4.8(dd, J=3, 8Hz, 1H), 4.45(m, 2H), 4.15(m, complex, 4H), 3.86(m, 3H), 3.6(m, complex, 1H), 3.45(m, complex, 1H), 1.35(2t, 6H).

## Example 4

Step 4)

(S)-9-(3-hydroxy-2-phosphonylmethoxypropyl)adenine (S-HPMPA;I)

A solution of diethyl-HPMPA(II;1.00 g, 0.00278 mol) in dry dimethylformamide (10 mL) was treated under nitrogen with bromotrimethylsilane (4 mL, 0.0278 mol). After the resulting mixture had been allowed to stand for 3 hours at room temperature, the volatile reactants and by-products were removed at 60°C and 5mm. The remaining residue was treated with water (5mL), followed by acetone. The solid that forms was collected, washed with acetone and recrystallized from boiling water/ ethanol to yield a white amorphous crystalline solid, mp. 243.5-244°C (sharp), which

proved to be a hemi-hydrate of I.

$^1$H NMR(DMSO-d6/$D_2O$, DMSO and $D_2O$ noise-suppressed) δ8.12(s, 1H), 8.10(s, 1H), 7.20(bs, 2H), 4.26(ABqd, J=3.5, 7, 14Hz, 2H), 3.75(bm, 5 lines, 1H), 3.59(m, 2H), 3.38(m, 1H), 3.30(bs, 2H). Analysis: ($C_9H_{14}N_5PO_5$ 1/2$H_2O$) C,H,N.

Example 5

Step 1)

N-2-(Diphenyl-4-(methoxyphenyl)methyl)-9-(2-hydroxy-3-(diphenyl-4-(methoxyphenyl)methoxy)propyl)guanine (IV)

A suspension of 9-(2,3-dihydroxy)propylguanine (5.0g, 0.022mol) in dry dimethylformamide (100mL) was treated with 30g (0.097mol) p-anisyldiphenylchloromethane, 40mL triethylamine, and 0.5g N,N-dimethylaminopyridine, and the resulting mixture was heated for 12h. at 80°C. The solution was then cooled, methanol (50mL) was added, and the volatile reactants and by-products were removed in vacuo at 70°C and 5mm. The residue was partitioned between ethyl acetate and water, and the combined ethyl acetate layers were dried

($MgSO_4$) and concentrated in vacuo. The dark oil remaining was chromatographed over silica gel eluting with 1:1 ethyl acetate/hexanes to yield 4.5g (27%) of the bis-monomethoxytrityl compound as a light orange foam, mp. 104-106°C (dec.), $^1H$ NMR($CDCl_3$, $Me_4Si$) δ 7.38(m, 6H), 7.26(m, 10H), 7.16(m, 8H), 6.78(d, J=7Hz, 2H), 6.72(d, J=7Hz, 2H), 6.23(s, 1H), 5.00(br s, 2H), 3.95(bm, 2H), 3.74(s, 3H), 3.14(bs, 1H), 2.88(bs, 1H); $^{13}C$ NMR(90MHz, $CDCl_3$, Me4Si) δ 159.97, 158.54, 158.05, 157.34, 153.55, 145.51, 144.04, 143.95, 140.11, 137.76, 136.28, 135.10, 130.05, 128.82, 128.12, 127.90, 127.74, 127.54, 126.87, 126.48, 114.99, 113.06, 112.85, 86.45, 70.45, 69.43, 67.79, 64.26, 55.04, 47.93; UV($CHCl_3$) λ max 285.5(e= 12,277); IR(KBr) 3210(broad), 3020, 3010, 2985, 1720, 1605(s), 1260(s), 700cm$^{-1}$; MS(EI) 768(M=1, 10); Analysis: ($C_{48}H_{43}N_5O_5$) C,H,N.

Example 6

Step 2)

N-2-(Diphenyl-4-(methoxyphenyl)methyl)-9-(2-diethylphosphonylmethoxy)-3-(diphenyl-4-(methoxyphenyl)methoxy)-propyl)guanine (III)

A solution of the bis-(monomethoxy)trityl DHPG from

Ex. 5 (3.0g, 0.0039mol) in dry THF (30mL) was treated in one batch with NaH (Aldrich, 0.311g, 0.0041mol, 60% by weight in oil). The solution was stirred for 15 minutes at room temperature, then treated with tosyloxymethyl diethylphosphonate (1.50g, 0.0046mol), and the resulting mixture stirred for 12h. at room temperature. Thin layer chromatographic analysis of the crude mixture revealed the absence of the starting alcohol (Rf 0.4 in 1:1 ethyl acetate/hexanes) and presence of a single polar product (Rf 0.1 in same). Methanol (10mL) was added, and the volatiles were removed in vacuo. The oil remaining was dissolved in ethyl acetate, applied to a column of silica gel (ca. 3X300cm) and eluted with pure ethyl acetate. The product was obtained in 40 fractions which were combined and concentrated in vacuo to yield 3.2gm (90%) of a colorless foam, mp.78-80$^{o}$C.

$^{1}$H NMR(360 MHz, $CDCl_3$, $Me_4Si$) δ 7.52(s, 1H), 7.41(d, J=7Hz, 4H), 7.27(m, 12H), 7.16(m, 8H), 6.80(d, J=7Hz, 2H), 6.72(d, J=7Hz, 2H), 6.21(s, 1H), 5.00(bs, 2H), 4.17(m, 1H), 4.07(q, J=7Hz, 1H), 3.98(q, J=7Hz, 2H), 3.80(m, 1H), 3.74(s, 3H), 3.71(s, 3H), 3.67(m, complex, 1H), 3.62(m, complex, 1H), 3.14(m, 1H), 1.25 (t, J=7Hz, 3H), 1.19(t, J=7Hz, 3H); 13C NMR(90MHz, $CDCl_3$, $Me_4Si$) δ 159.71, 158.52, 157.40, 153.41,

145.73, 143.48, 140.02, 137.91, 136.39, 134.87, 130.04, 128.79, 128.10, 127.86, 127.69, 127.55, 127.40, 126.86, 126.31, 114.65, 113.03, 112.71, 86.45, 79.25($J_{C-O-C-P}$ = 9Hz), 70.28, 67.59, 64.09, ($J_{C-P}$ = 166.5Hz), 62.10, 54.98, 44.42, 16.42; UV($CHCl_3$) λmax 285.5(e= 12,602); Analysis. ($C_{53}H_{54}N_5O_8P$) C,H.

Example 7

Steps 3) & 4)

(S)-9-(3-hydroxy-2-phosphonylmethoxypropyl)guanine (S-HPMPG)

A solution of the bis-(monomethoxy)trityl diethyl HPMPG (1.5g, 0.0016mol) in 80% aqueous acetic acid (50mL) was heated gently on a steam bath for 1/2h. Thin layer chromatographic analysis indicated that the starting phosphonate was absent, and that tritanol by-product and diethyl-HPMPG were the only compounds present (Rf 1.0 and 0.2 in 10% methanol/methylene chloride respectively). The mixture was evaporated to dryness, co-evaporated with absolute ethanol (4x100mL), and finally with toluene (2x100mL). The yellow solid obtained was triturated with ethyl acetate/toluene (1:1, 3x100mL), and TLC of the ehtyl acetate/toluene extracts indicated that only tritanol was present. The solid material remaining after the trituration

was dried by evaporating with toluene, and further dried in vacuo for two hours. The crude diethyl-HPMPG thus obtained (mp. 87-90°C) was treated with 5mL bromotrimethylsilane in dry dimethylformamide (10mL). The resulting light yellow mixture was allowed to stand at room temperature for 5 hours. The volatile reactants and by-products were then removed in vacuo, water (5mL) followed by acetone (5mL) were added, and the turbid solution kept at -20°C for 1h. The solid that had formed was collected by suction filtration, washed with acetone, and recrystallized from water/acetone to yield an off-white solid, mp. 185-190°C (dec.).

$^{1}H$ NMR(360MHz, DMSO-d6) δ 7.72(s, 1H), 6.47(bs, 2H), 4.15(B part, ABq, J= 3.5, 14Hz, 1H), 3.98(A part, ABq, J=7, 14Hz, 1H), 3.67(m, complex, 1H), 3.62(m, 5 lines, 2H), 3.37(m, complex, 2H); $^{13}C$ NMR(90MHz, DMSO-d6) δ 156.72 153.67, 151.31, 138.25, 115.83, 80.45($J_{C-O-C-P}$= 10Hz), 69.86, 66.39, 64.61($J_{C-P}$= 166Hz), 43.81.

Example 8

Preparation of starting 9-(2,3-Dihydroxypropyl)guanine

A.

2',3'-O-Isopropylidene-6-O-benzyl-(S)-9-(2',3'-dihydroxy)-propyl)guanine

A 250 mL 3-necked round bottomed flask fitted with a gas inlet, was oven dried, flushed with argon, and charged with sodium hydride (1.82g, 0.045mol, 60% by weight in oil). The sodium hydride was washed twice with 50mL of dry pentane ($CaH_2$), once with dry THF (Na/benzophenone), and covered with dry dimethylformamide (250mL, distilled from $P_2O_5$). 2-Amino-6-benzyloxypurine* (10.00g, 0.041mol, prepared from 2-aminopurin-6-yl-trimethylammonium chloride)** was added in one batch, and the solution heated at 60°C for 1h. Isopropylidene-D-glycerol-γ-tosylate (11.86g, 0.041mol, Fluka) was then added in one batch, followed by a catalytic amount (1g) of sodium iodide, and the resulting mixture heated for 12h at 60°C. The solution was then cooled and the volatile reactants and by-products removed under reduced pressure. Thin layer chromatographic analysis of the crude mixture revealed the presence of the N-9 isomer(Rf 0.7 in 10% methanol/methylene chloride) and the N-7 isomer (Rf 0.3 in same). Chromatography over silica gel eluting with ethyl acetate gave 10g of the N-9 isomer as a gum, and 2g of the crystalline N-7 isomer, mp. 184-186C (80% overall yield, 5:1 ratio of N-9/N-7). Characterization of each isomer was as follows:

*MacCoss, M.; Chen, A.; Tolman, R.L. Tetetrahedron Lett., 26, 1:15 (1985)

**Kiburis, J.; Lister, J.H., J. Chem. Soc., C, 3942 (1971).

N-9: $^{1}$H NMR (360 MHz, $CDCl_3$, $Me_4Si$) δ 7.66(s, 1H), 7.47(d, J=1 Hz, 2H), 7.30 (m, complex, 3H), 5.52(s, 2H), 4.93(bs, 2H), 4.39(m, complex, 1H), 4.19(dd, A part, ABq, J=4, 14Hz, 1H), 4.08(dd, B part, ABq, J=4Hz, 1H), 4.01(dd, J=6, 8Hz, 1H), 3.66(dd, J=6, 8Hz, 1H), 1.32(s, 3H), 1.28(s, 3H); $^{13}$C NMR($CDCl_3$, $Me_4Si$) δ 160.47, 158.98, 153,78, 139.85, 135.93, 127.80, 127.57, 127.39, 114.44, 109.37, 73.44, 67.47, 65.88, 49.42, 44.66, 26.12, 24.66; a gated heteronuclear decoupling experiment ($^{13}$C) revealed that C-5 is coupled only to the proton at C-8 (J=10Hz), whereas C-4 was coupled to the proton at C-8 (J=12Hz) and the methylene protons at C-1 (J=4Hz); MS(CI) 356(M+H, 100), 266(M-benzyl, 18), 91($CH_2$phenyl, 95).

N-7: $^{1}$H NMR (360MHz, $CDCl_3$, Me4Si) δ 7.80(s, 1H), 7.35(m, complex, 5H), 5.46(s, 2H), 5.08(bs, 2H), 4.31(2 overlapping m, complex, 2H), 4.20(dd, J=7, 14Hz, 1H), 3.82(dd, J=6, 8Hz, 1H), 3.52(dd, J=6, 8Hz, 1H), 1.26(s, 3H), 1.24(s, 3H); $^{13}$C NMR(90MHz, $CDCl_3$, $Me_4Si$) δ 163.80, 159.10, 156.76, 145.63, 135.83, 128.47, 128.25, 128.07, 109.84, 106.91, 74.20, 67.99, 65.94, 49.00, 26.47, 25.12; a gated heteronuclear decoupling experiment was performed (as for the N-9 isomer) which indicated that C-4 coupled only to the proton at C-8 (J=10Hz), whereas C-5 was coupled to the proton at C-8 (J=12Hz), and also to the methylene protons at C-1' (J=4Hz);

Analysis: $(C_{18}H_{21}N_5O_3)$ C,H,N.

B. 9-(2,3-Dihydroxypropyl)guanine

A solution of 2',3'-O-isopropylidene-6-O-benzyl-(S)-9-(2',3'-dihydroxypropyl)guanine (5.0g, 0.0139mol) in 80% aqueous acetic acid (80mL) was heated on a steam bath for 1h. The volatile reactants by-products and solvent were then removed in vacuo, and from the residue remaining were evaporated four 100mL volumes of absolute ethanol followed by two 100mL volumes of toluene. The white solid material obtained was recrystallized from water and dried at 5mm for 12h. to yield 2.8g (89%) of 9-(S)-(2,3-dihydroxypropyl)guanine as a white solid, mp. above 260°C.

$^1$H NMR(360MHz, DMSO-d6) δ 10.57(s, 1H), 7.58(s, 1H), 6.44(bs, 2H), 5.05(d, J=5Hz, 1H), 4.77(t, J=5Hz, 1H), 4.07(d, J=11Hz, 1H), 3.77(2 overlapping m, complex, 2H), 3.35(m, complex, 1H), 3.27(m, complex, 1H); $^{13}$C NMR(90MHz, DMSO-d6) δ 156.90, 153.47, 151.32, 138.36, 116.38, 69.77, 63.51, 46.10; UV (0.1N aq. HCl) λ max 253(e=12,305), λ max 272(e=8,495); (0.1N aq. NaOH) λ max 256(e=10,096), λ max 267 (e=10,614); $[\alpha]_D^{25} = -45°$, $[\alpha]_{456}^{25}=-54°$ (c=0.5, DMSO);

IR(KBr) 3180 (b, s), 3100(s), 1695, 1650, 1605 $cm^{-1}$;
Analysis: ($C_8H_{11}N_5O_3$) C,H,N.

6-(2-methoxyethoxy-9-(2,3-dihydroxypropyl)guanine was prepared by following substantially the procedure described in Example A and deprotecting as in Example B except that 6-(2-methoxyethoxy)guanine* was used in the place of 2-amino-6-benzyloxypurine.

*(Keillberg, J.; Liljenberg, M.; Johansson, N.G., Tetrahedron Lett., 27, 877 (1986))

What is claimed is:

1. A process for preparing a compound of Formula I

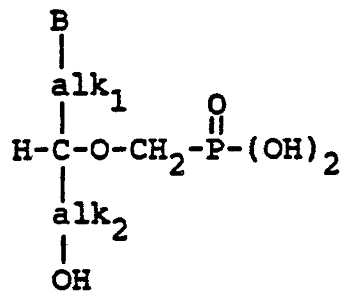

I

wherein $alk_1$ and $alk_2$ ,independently, are selected from $C_{1-4}$ alkylene; and

B is a purine base selected from adenin-9-yl and guanin-9-yl,

which comprises the consecutive steps of:

a) reacting the diol starting material V

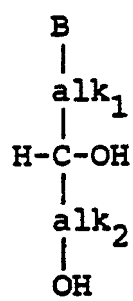

V

with a protective group precursor compound of formula PG-X, wherein PG is a selective protecting group having steric bulk and X is a leaving group moiety selected from halide groups, to give the di-protecting group-intermediate IV

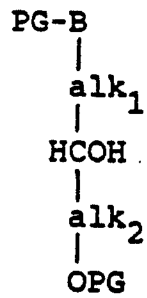

IV ;

b) treating IV with an alkali metal hydride and then with VI

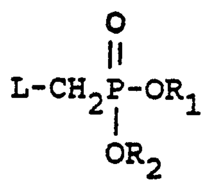

VI

wherein $R_1$ and $R_2$, independently, are selected from $C_{1-6}$ alkyl and L is an organic leaving group, to yield the dialkylphosphonylmethyl intermediate III

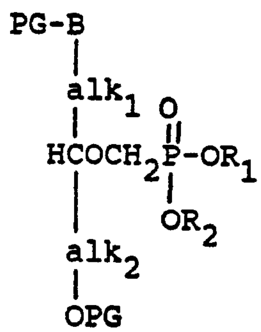

III ;

c) removing the protecting groups PG from III by a method selected from heating III in acidic media and mild

hydrogenolysis of III to give the de-protected dialkylphosphonylmethyl intermediate II

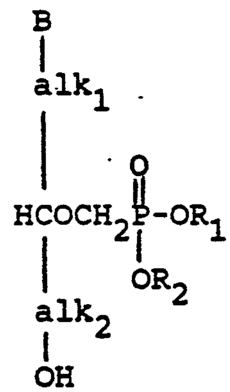

II ; and

d) cleaving the phosphonate ester alkyl groups $R_1$ and $R_2$ from II by the halotrialkylsilane mediated ester dealkylation of II followed by the addition of water to give the desired product I.

2. The process of claim 1 wherein the protecting group PG of PG-X is selected from the group consisting of triphenylmethyl, p-methoxyphenyldiphenylmethyl and di-(p-methoxyphenyl)phenylmethyl and X is a halide group selected from chloride and bromide.

3. The process of claim 1 wherein the metal hydride used in step b) is an alkali metal hydride, preferably sodium hydride.

4. The process of claim 1 wherein VI in step b) is selected from tosyloxymethyl dialkylphosphonate and triflyloxymethyl dialkylphosphonate.

5. The process of claim 1 wherein the protecting group removal method of step c) is heating in aqueous acid, preferably in aqueous acetic acid.

6. The process of claim 1 wherein the cleavage of step d) consists of reacting the dialkyl phosphonate intermediate, II, with a halotrimethylsilane reagent followed by the addition of water to effect the conversion to the phosphonic acid, I.

7. The process of claim 2 wherein PG is triphenylmethyl and X is chloride.

8. The process of claim 6 wherein the halotrialkylsilane reagent is halotrimethylsilane with halo being bromo and iodo and wherein there is included after reaction with the halotrimethylsilane and before the addition of water the further step of removing volatile reactants, by-products and solvents.

9. The process of claim 1 wherein $alk_1$ and $alk_2$ are mono-methylene.

10. A process for preparing (S)-9-(3-hydroxy-2-phosphonylmethoxypropyl)B, wherein B is a purine base selected from adenin-9-yl or guanin-9-yl, which comprises the consecutive steps of:

a) reacting (S)-9-(2,3-dihydroxypropyl)B with chlorotriphenylmethane to give N,3'-O-bis-triphenylmethyl-(S)-9-(2',3'-dihydroxypropyl)B;

b) treating N,3'-O-bis-triphenylmethyl-(S)-9-(2',3'-dihydroxypropyl)B with sodium hydride and then with tosyloxymethyl diethylphosphonate to obtain N,3'-O-bis-triphenylmethyl-2'-O-(diethylphosphonyl methyl)-(S)-9-(2',3'-dihydroxypropyl)B;

c) heating N,3'-O-bis-triphenylmethyl-2'-O-(diethylphosphonylmethyl)-(S)-9-(2',3'-dihydroxypropyl)B in aqueous acetic acid to obtain (2'-O-diethylphosphonylmethyl)-(S)-9-(2',3'-dihydroxypropyl)B; and

d) treating (2'-O-diethylphosphonylmethyl)-(S)-9-(2',3'-dihydroxypropyl)B with bromotrimethylsilane and then with water followed by acetone to give 2'-O-phosphonylmethyl-(S)-9-(2',3'- dihydroxypropyl)B.

11. The compound of formula III

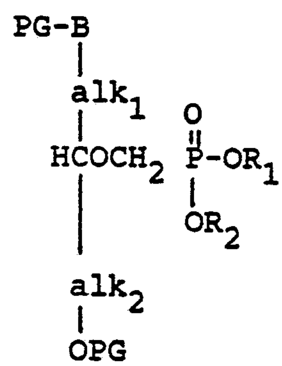

III

wherein $alk_1$ and $alk_2$ are independently selected from $C_{1-4}$ alkylene;

$R_1$ and $R_2$ are independently selected from $C_{1-6}$ alkyl;

B is adenin-9-yl or guanin-9-yl; and

PG is selected from the group consisting of triphenylmethyl, p-methyoxyphenyl-diphenylmethyl and di-(p-methoxyphenyl)phenylmethyl.

12. The compound of formula IV

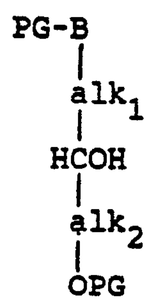

IV

wherein $alk_1$ and $alk_2$ are independently selected from $C_{1-4}$ alkylene;

B is adenin-9-yl or guanin-9-yl; and

PG is selected from the group consisting of triphenylmethyl, p-methoxyphenyldiphenylmethyl and di-(p-methoxyphenyl)phenylmethyl.

European Patent Office

# EUROPEAN SEARCH REPORT

Application Number

EP 87 11 6995

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | J. MED. CHEM., vol. 29, 1986, pages 671-675, American Chemical Society; E.J. PRISBE et al.: "Synthesis and antiherpes virus activity of phosphate and phosphonate derivatives of 9-[(1,3-dihydroxy-2-propoxy)methyl]guanine" * Pages 672-673, "Chemistry"; page 675 * | 1-10 | C 07 F 9/65<br>C 07 D 473/18<br>C 07 D 473/34 |
| D,Y | FR-A-2 539 132 (CESKOSLOVENSKA AKADEMIC VED) * Page 2, line 5 - page 4, line 8; examples 2-7 * | 1-10 | |
| X | J. ORG. CHEM., vol. 50, no. 6, 1985, pages 755-759, American Chemical Society; J.C. MARTIN et al.: "Synthesis of 9-(4-hydroxy-2-oxobutyl)guanine, 9-(2,4-dihydroxybutyl)guanine, and related acyclic nucleoside analogues" * Page 756, scheme I; pages 757-758, compound 15e * | 12 | |
| | | | TECHNICAL FIELDS SEARCHED (Int. Cl.4) |
| | | | C 07 F 9/00<br>C 07 D 473/00 |

The present search report has been drawn up for all claims

| Place of search | Date of completion of the search | Examiner |
|---|---|---|
| THE HAGUE | 29-03-1988 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document